# EUROPEAN PATENT SPECIFICATION

(11) **EP 3 640 906 B1**
(45) Date of publication and mention of the grant of the patent: **27.09.2023**
(21) Application number: 18746312.0
(22) Date of filing: 07.06.2018
(51) Int. Cl.: G08B 21/04, A61B 5/00, A61B 5/11

(54) **COVERING DEVICE AND MONITORING SYSTEM FOR PRESENCE AND MOTION RECOGNITION**
DISPOSIÇÃO DE COBERTURA E SISTEMA DE MONITORIZAÇÃO PARA RECONHECIMENTO DE PRESENÇA E MOVIMENTO
AGENCEMENT DE COUVERTURE ET SYSTÈME DE SURVEILLANCE POUR RECONNAISSANCE DE PRÉSENCE ET DE MOUVEMENT

(30) Priority: 13.06.2017 PT 2017110143
(43) Date of publication of application: 22.04.2020
(73) Proprietor: Flexitex - Fabrica De Tecidos SA, 3700-257 São João Da Madeira (PT)
(72) Inventor: DA S. RODRIGUES ANDRADE, Antonio Jose, 3800-735 Aveiro (PT); OLIVEIRA FREITAS, Diana Rosa, 4520-154 Santa Maria Da Feira (PT); RODRIGUES CHAVES FERREIRA, Paulo Alexandre, 1000-138 Lisboa (PT); GUIMARAES PIMENTA, Nelson Rafael, 1000-138 Lisboa (PT)
(74) Representative: Liebl, Thomas
(86) International application number: PCT/PT2018/000011
(87) International publication number: WO 2018/231080

(56) References cited:
- WO-A1-2016/156779
- GB-A- 2 464 965
- US-A1- 2017 042 340

## Description

The present invention refers to the field of cover dispositions for objects such as for example beds, and recognition of the presence and movement of individuals upon a respective support surface.

The present invention further refers to a monitoring system according to the present invention.

### Background of the invention

There are known several documents in prior art relating to dispositions for detecting movement of people in beds, including as monitoring means for application in hospitals and premises for accommodating senior individuals.

In particular, it is known using a disposition of the type pressure-operated electrical switch. Typically, said electrical switch is provided by the superposition of two conductors that result in contact when pressured together by the weight of an individual.

Documents US 4,263,586, US 4,295,133, disclose solutions related with this type of switch disposition.

Documents US 4,484,043 and US 6,696,653 B1 disclose a disposition of parallel conducting strips where there is a superposition between conductors that provided on a common vertical alignment along their extension.

Document EP 0671145 B1 discloses a similar disposition comprising a planar member underneath, a planar member over that deflects under the action of the weight of an individual and a sensor means placed between said planar members. This solution does not consider the measurement of variations of electrical conductivity nor provides an integration of a conducting textile disposition with a textile support.

Document US 6,987,232 B2 discloses a sensor and a method for detecting the movement of a patient, whereby the sensor uses a linearly configured resistive leader with resistors spaced apart as a means for determining at least approximately the location of a patient on the sensor, as well as an approximate length of the sensor that is compressed by the patient. The sensor comprises a top non-conducting member and a bottom non-conducting member.

Document US 7,396,331 B2 discloses a system and process for non-invasive collection and analysis of physiological signals.

Document WO 2008/104918 A1 discloses a system and method for obtaining physiologic data from a patient. Said system comprises at least one elastically deformable member, whereby said member is deformable by the patient lying in a bed. The method includes acquisition of deformation data of said member and the determination of physiological data of said patient based upon said deformation data.

Document WO 2009/124397 A2 discloses a signalling device for detecting the presence of an object, comprising first and second spacing elements positions in top and bottom layers, so that when forces are externally applied, the spacing elements collapse and there is established a conducting path between said first and second conductors.

Document WO 2013/003963 A1 discloses a device for detecting positional changes of people lying in beds. Said device comprises at least one elongated bridge for support of mattress and for transmission of the supported load. Said bridge comprises at least one plate with at least one section that is configured resistant to flexion. In the path of transmission of force between the load support and the support device there is disposed at least one sensor for detecting a measurement variable that can be influence by the supported load. This solution does not consider either measurement of variations of electric conductibility nor provides an integration of a conducting textile disposition with a textile support

Document US 2017/042340 A1 discloses an occupancy detection arrangement for furniture such as a bed, by means of capacitance detection.

Document WO 2016/156779 A1 discloses an occupancy monitoring device for detecting movement of a person on an object such as a bed, by means of capacitance detection.

None of the documents in prior art discloses an integration of several electrical components in a textile cover disposition adapted for being applied upon an object for supporting individuals, such as for example a mattress. There is therefore the need for a cover disposition of this type that provides more reliable and efficient electrical connections, as well as a quick and ergonomic installation, without the need for particular care with components and electrical connections.

### Description of the invention

The objective of the present invention is to provide a cover disposition for monitoring of an individual in a respective support surface, in particular on a support surface that comprises a textile material support, and so that provides a better integration of components and electric connections in said cover disposition and a more reliable monitoring.

In particular, the objective of the present invention is to provide a more reliable and efficient association of a plurality of textile conductors with a respective textile material support, and with an object adapted for supporting individuals, so as to provide a reliable measurement by said textile conductors of solicitations upon said textile material support.

The objective above is solved according to the present invention by means of a cover disposition according to claim 1.

It is herewith provided a textile cover disposition comprising several electrical means, adapted for direct and quick application, without great installation requirements and with smaller risks of bad functioning or low measurement reliability.

It is preferred when the cover disposition presents a conducting textile disposition provided on a top part and at least one of retention means of cover disposition and closing means of parts of said cover disposition, so that said cover disposition can be applied upon an object for supporting individuals, such as for example mattress, and removed from said object, without the need of establishing or disconnecting electrical connections.

It is preferred when said textile material support is provided as a cover element of at least one surface adapted for an individual to laydown, sit or layover, of an object for supporting individuals, including piece of furniture, such as for example chair, or support for accommodating individuals, such as for example the mattress of a bed.

A related objective, not being part of the present invention, is to provide a cover disposition for monitoring presenting more reliable and efficient electricity conducting connections between a conducting textile disposition and a respective control device.

In particular, it is an objective, not being part of the present invention, to provide technical conditions for a reliable measurement of at least one relevant electricity parameter of said conducting textiles, as result of solicitations, in particular forces generally along the direction of the gravity force, upon an associated textile material surface.

The aforementioned objective, not being part of the present invention, is solved by means of a cover disposition whereby the energy conducting disposition between said conducting textile disposition and said control device, comprises:
- first connection means adapted for conducting connection of a first and second end of said conducting textile disposition;
- an energy conducting disposition adapted for connection of said first connection means to second connection means;
- second connection means adapted for conducting connection of said energy conducting disposition to said control device,
whereby both said first and second connection means are provided as pieces comprising respective conducting metallic parts, and said first connection means are adapted so that can be fixed to said conducting textiles by means of a clamping/ crushing type of connection.

A related objective not being part of the present invention is to provide a monitoring disposition that provides a more reliable and efficient monitoring of the presence and movement of an individual on a respective object for supporting individuals, such as for example the mattress of a bed.

In particular, the monitoring disposition according to the present invention comprises a textile material support that presents at least one, preferentially a plurality of conducting textile wires, and at least one control device adapted so that can apply an electric tension in successive moments, measuring at least one electric conductibility parameter, comparing with at least one previously measure value in each one of said conducting textile wires, so as to identify a variation of said electric conductibility parameter.

A related objective not being part of the present invention is to provide a cover disposition comprising a conducting textile disposition, an energy conducting disposition and a control device provided in operative connection.

It is preferred when the control device is provided on a lateral part of said textile material support an in the proximity of an energy source, preferentially in the proximity of an opening region provided by support closing means.

It is preferred when said energy conducting disposition is retained along at least part of its extension by a plurality of retention elements associated with said textile material support and configured in a shape of sheath, chain-link, or similar, so as to maintain the plurality of conducting yarns contained in said energy conducting disposition in relative proximity to each other along at least part of respective extension.

It is preferred when said conducting textile disposition comprises at least one, preferentially a plurality of conducting textiles developing along an elongated extension, and comprising a plurality of conducting particles provided so that provide conduction of electrical current along the elongated extension of said conducting textiles.

It is preferred when said conducting textiles are provided as a structure of textile material that presents a distribution of metallic particles provided with a given density and alignment so that provide electric energy conducting properties to said textile structure that vary according to the extension of said conducting textiles relative to a non-stretched situation, whereby said textile structure is preferentially of the type textile yarn, textile string, textile strip, or similar.

It is preferred when said textile support comprises:
- a first textile support presenting a plurality of first conducting textiles disposed at least approximately parallel to each other along a first extension, preferentially along a longitudinal extension, and
- a second textile support presenting a plurality of second conducting textiles disposed at least approximately parallel to each other,
whereby said second textile support is provided superposed to said first textile support, so that said first and second textile conductors provide a disposition of type jacquard tissue, or similar, upon at least part of the support surface of said textile support.

It is preferred when said first and second support elements are disposed so that said first and second conducting textiles configure a general shape of the type jacquard tissue and can establish electric conducting contact in given intersection regions in certain intersection regions in superposition in the case of a force being applied with a vertical component upon said textile material support.

It is preferred when said textile material support comprises a first support element presenting a conducting textile disposition with first conducting textiles developing along a first direction, and a second support element presenting a textile disposition with second conducting textiles developing along a second direction orthogonal with relation to said first direction, whereby said first and second conducting textiles are provided so that can establish an electrical conducting contact between intersection regions as a result of the application of a force along the direction of the gravity force upon the surface of the top part of textile material support.

It is preferred when the cover disposition presents an electric insulating element between first and second support elements of said textile material support, whereby said electric insulating element presents a shape similar to the superposed top parts of said first and second support elements, there is provided electric insulating material, such as for example a synthetic material, with a foam-like structure or similar, and presents a plurality of through-holes provided in regions corresponding to the intersecting regions in superposition of said first and second conducting textiles, so that provide an electric contact between these when said cover disposition is submitted to a load along the direction of the gravity force.

It is preferred when the cover disposition presents an electric insulating element disposed between first and second support elements of said textile material support, presenting a plurality of through-holes, whereby each one of said through-holes presents a pressure-switch disposition that comprises:
- a first element in electric conducting material, with a foam-like structure or similar, presenting a thickness of up to 5 mm,
- a distancing element, provided in a electric non-conducting material, preferentially with a foam-like structure, configured for example with ring-like shape or similar, presenting a height of up to 5 mm, and
- preferentially, a second layer of conducting foam, in electric conducting material, with a foam-like structure or similar, presenting a thickness of up to 5 mm.

The present invention shall hereinafter be explained in greater detail based upon preferred embodiments and in the attached Figures.

The Figures show, in simplified schematic representations:
- Figure 1:: diagram of a first embodiment of a monitoring unit (1) of a monitoring disposition;
- Figure 2:: diagram of a plurality of monitoring units (1) included in a monitoring disposition;
- Figure 3:: diagram of a preferred embodiment of conducting textile disposition (31, 32) in a textile material support (2) in a monitoring disposition (1);
- Figure 4:: diagram of a preferred embodiment of conducting textile disposition (3) in a monitoring disposition (1);
- Figure 5:: diagram of a preferred embodiment of a conducting textile disposition (3) of textile material support (2) in a monitoring disposition (1);
- Figure 6:: diagram of a preferred embodiment of a conducting textile disposition (3) of textile material support (2) in a monitoring disposition (1);
- Figure 7:: diagram of a preferred embodiment of configuration of textile material support (2) in a monitoring disposition (1), in an open position;
- Figure 8:: diagram of a preferred embodiment of configuration of textile material support (2) in a monitoring disposition (1), in a closed position;
- Figure 9:: diagram of an electricity conducting disposition between a conducting textile disposition (3) and a respective control device (7) in a monitoring system not being part of the invention, with first connection means (4) open;
- Figure 10:: diagram of an electricity conducting disposition between a conducting textile disposition (3) and a respective control device (7) in a monitoring system not being part of the invention, with first connection means (4) closed;
- Figure 11:: assembly diagram of a invention;monitoring disposition (1) not being part of the
- Figure 12:: assembly diagram of a monitoring disposition (1) not being part of the invention;
- Figure 13:: assembly diagram of a monitoring disposition (1) not being part of the invention;
- Figure 14:: assembly diagram of a monitoring disposition (1) not being part of the invention;
- Figure 15:: detail of assembly of a monitoring disposition (1) not being part of the invention;
- Figure 16:: diagram of a monitoring disposition (1) not being part of the invention.

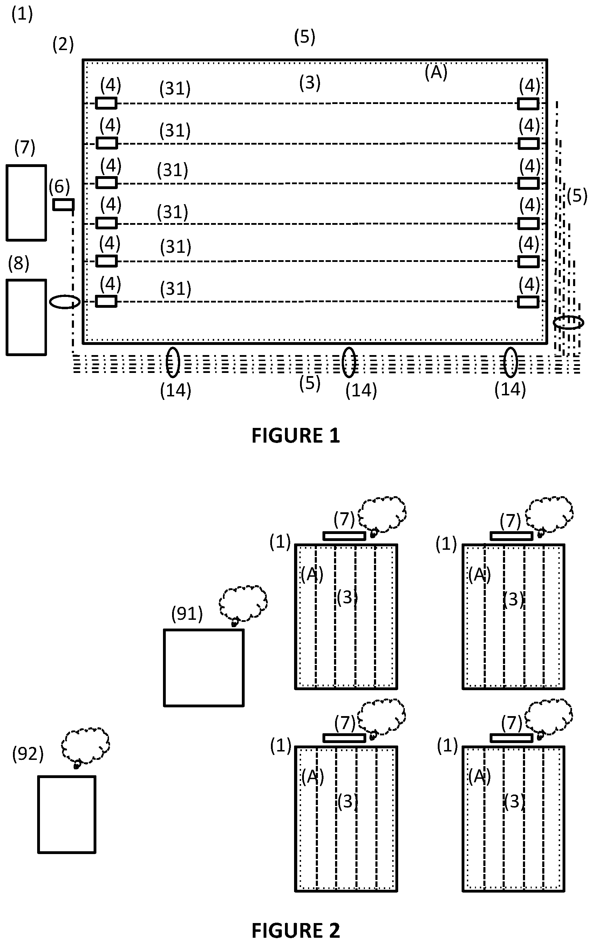

**Figure** 1 represents a preferred embodiment of a monitoring system according to the present invention, comprising a conducting textiles disposition (3) associated with a textile material support (2) and provided in conducting connection of electric energy with a control device (7).

The conducting textile disposition (3) comprises a plurality of conducting textiles (31) disposed along a substantially parallel direction relative to each other, and at a distance (D) between each other, whereby an electric tension can be applied between first and second ends of each one of said conducting textiles (31).

According to a first aspect, said conducting textiles (31) comprise a plurality of conducting particles provided along the elongated extension of said conducting textiles. Moreover, said conducting textiles (31) are adapted so as to provide a measurable variation of electric conductibility when submitted to stretching, including as a result of deflection along a direction transversal to respective elongated extension, in at least one region of respective elongated extension, so as to generate a variation of at least of distance and relative orientation between conducting particles provided on said conducting textiles (31) along the respective elongated extension.

In particular, said conducting textiles disposition (3) comprises a plurality of first conducting textiles (31) generally disposed along a first direction of said textile material support (2) and adapted so as to provide a measurable variation of electric conductibility when submitted to a force presenting a component perpendicular to the support surface provided by said textile material support (2).

Said conducting textiles (31, 32) are provided as a textile material structure that presents a distribution of metallic particles provided with a given density and alignment so as to provide electric energy conducting properties to said textile structure that vary according to the stretching of said conducting textiles (31, 32) relative to a non-stretched condition, whereby said textile structure is preferentially of the type textile yarn, textile string, textile strip, or similar. The application of an electric tension enables therefore measuring at least one parameter relating to the electric conductibility, for example the electric resistance, of each conducting textile (31), so that variations of said parameter in time provide an indication of load variations thereupon.

**Figure 2** represents a non-inventive system comprising a plurality of monitoring units (1), for example beds, presenting respective control devices (7) provided in short range data connection, for example by means of Bluetooth, or similar, with a first remote data means (91) that can communicate data received with a second remote data means (92), for example by means of WIFI, or similar.

It is further preferred when the process of operation of a system further comprises the presentation of measured data, including data relating to types of events, respective time of occurrence, determined based upon periodic measurement of variations of at least one parameter of electric conductibility.

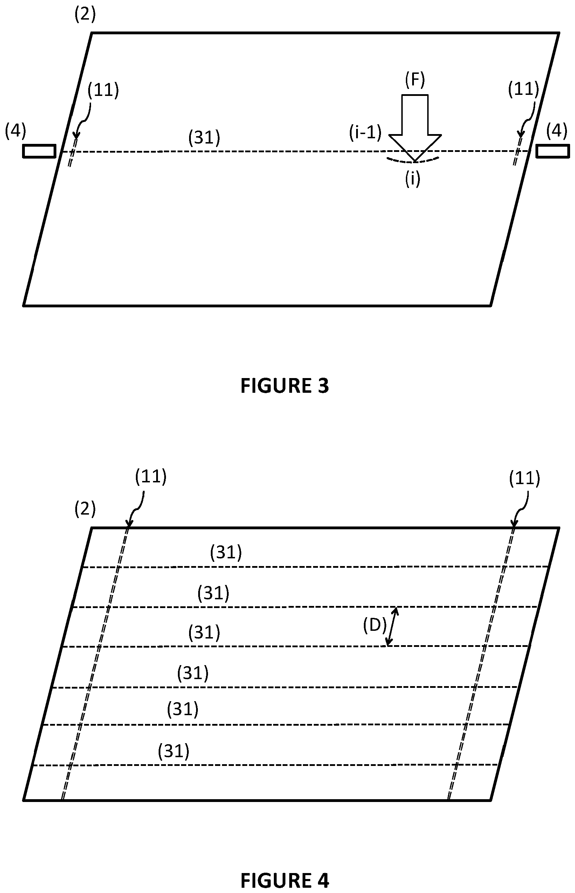

As schematically represented in **Figure 3**, according to an inventive aspect, said control device (7) is associated to an energy source (8), and adapted so that it can regulate by means of said control device (7), the realization of successive cycles i, i+1, ..., whereby each cycle comprises the application of a previously defined electric tension to each one of the conducting textiles (31) of said conducting textiles disposition (3), the measurement of at least one energy parameter related with the electric conductibility through said conducting textiles (31) and the assessment of an eventual variation in the measured value of said energy parameter with relation to at least one previous measurement.

Moreover, each monitoring cycle further includes the step of determining, based upon the value measured in a cycle i, a correspondence between at least one of:
- a variation previously measure in at least one cycle i-1 to i-n, and
- a plurality of previously measured variations in at least two cycles i-1 to i-n,
and a given type of event, including at least one of:
- entry and exit of an individual in contact with said conducting textile disposition (3);
- movement of an individual in contact with said conducting textile disposition (3).

According to a preferred embodiment, each monitoring cycle further includes determining a relative location on said conducting textile disposition (3), of a given interval of measured variations for at least one parameter associated with the electric conductibility of said conducting textile disposition (3).

Moreover, it is preferred when the control device (7) is provided so that can determine a relation between variations above a given threshold and the location of variations of at least one parameter associated to the electric conductibility on said conducting textile disposition (3), and respective evolution in time.

According to a preferred embodiment, said control device (7) is provided so that controls the application of an electric tension successively to the ends of each one of a plurality of conducting textiles (31), with a periodicity comprised between 0,001 s and 0,1 s, in ongoing manner in time.

According to a preferred embodiment (see **Figure 4**), both end regions of said conducting textiles (31) in the proximity of the limits of the top part are additionally fixed to the textile material support (2) by means of fixation means (11), provided so as to prevent the possibility of substantial displacement along the elongated extension thereof with relation to said textile material support (2). It is thereby ensure that the stretching of said conducting textiles (31) provides a reliable indication of the solicitation by a load applied upon said textile material support (2).

It is preferred when said conducting textiles are provided at distances (D) between each other of at least 10 mm, preferentially at least 20 mm, and at most 50 mm, preferentially at most 30 mm.

According to an inventive aspect, said textile material support (2) is provided so that it can be applied upon said object (A) for supporting of individuals, for example a mattress, so that said conducting textiles disposition (3) is substantially retained upon said object (A), at least with relation to movements along a longitudinal extension of said object (A). Moreover, said control device (7) and electric connections between the latter and said conducting textiles disposition (3) are associated with said textile material support (2), preferentially retained thereon, and said electric connections are provided so that the placement of said textile material support (2) on said object (A) does not require establishing any electric connection, and the removal of said textile material support (2) does not require disconnecting any electric connection.

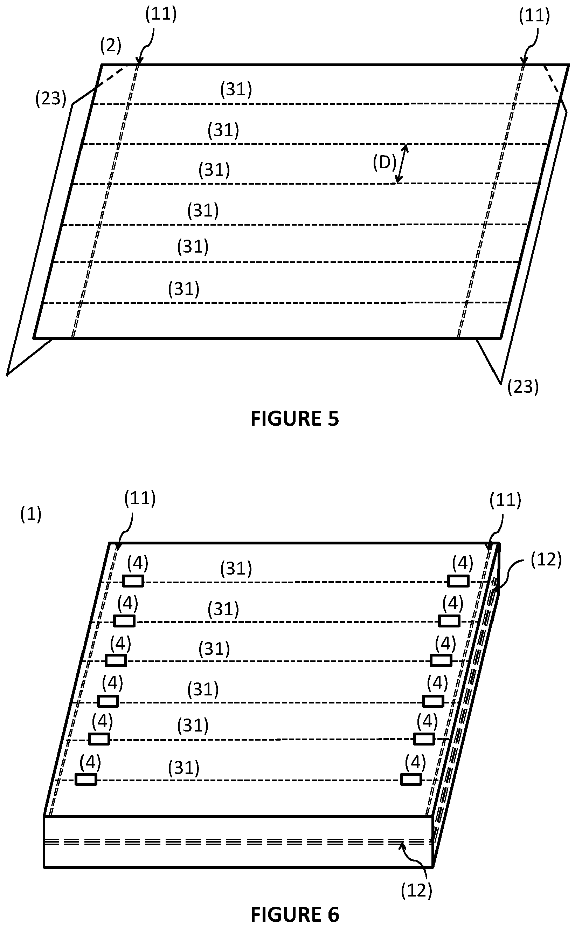

In the case of a first embodiment (1) represented in **Figure 5**, the textile material support (2) is provided with retention means (23) provided so that they can retain said textile material support (2) in a substantially fixed position with relation to said object (A).

**Figure 6** represents a second embodiment of a monitoring unit (1) where the textile material support (2) presents an at least partially closed general configuration that can collect at least partially said object (A) inside thereof underneath said top part (21, 22) and in a substantially fixed position with relation to said textile material support (2).

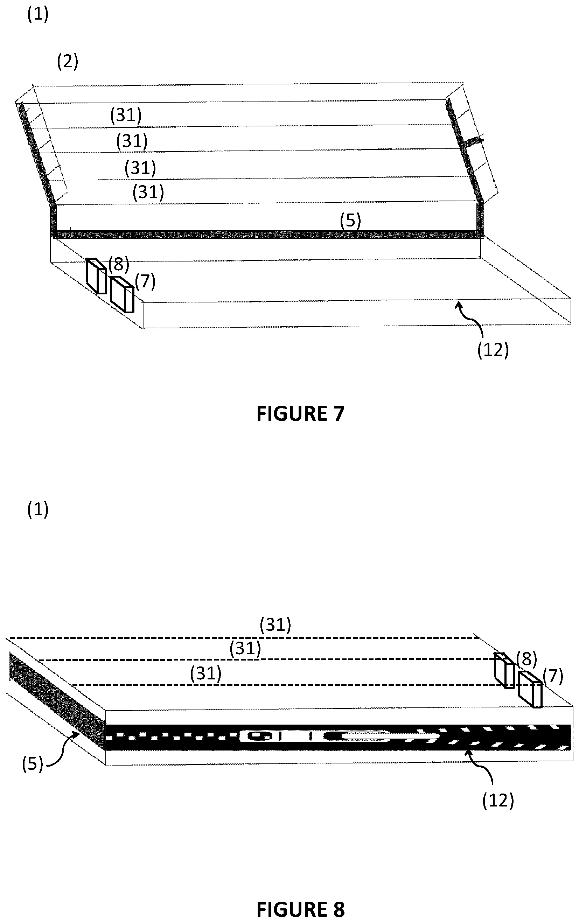

**Figures 7** and **8** represent a monitoring unit (1) corresponding to the embodiment represented in Figure 6, with a general configuration of box type, or envelope, in positions of open and closed, respectively.

In particular, it is preferred when said textile material support (2) presents support connection means (12) adapted so as to provide connection between at least two of said parts of textile material support (2), such as for example of said top part (21, 22) to the remaining parts, along at least part of a perimeter region of textile material support (2), so that said textile material support (2) can be opened, applied as envelope of a support element, and closed, and vice-versa.

It is further preferred when said textile material support (2) is provided with a general shape so as to confine said object (A) for supporting individuals inside thereof, and when said control device (7), energy source (8) and all the energy conducting connections (4, 5, 6) between said conducting textile disposition (3) and said control device (7) are provided inside of the confinement provided by said textile material support (2), so that each monitoring unit (1) does not present wire connections to the exterior, for example to external devices.

This type of configuration of monitoring unit (1) provides a rapid application upon an object (A), such as for example a mattress, without the need to carryout an installation of electric components or of establishing electric connection between thereof.

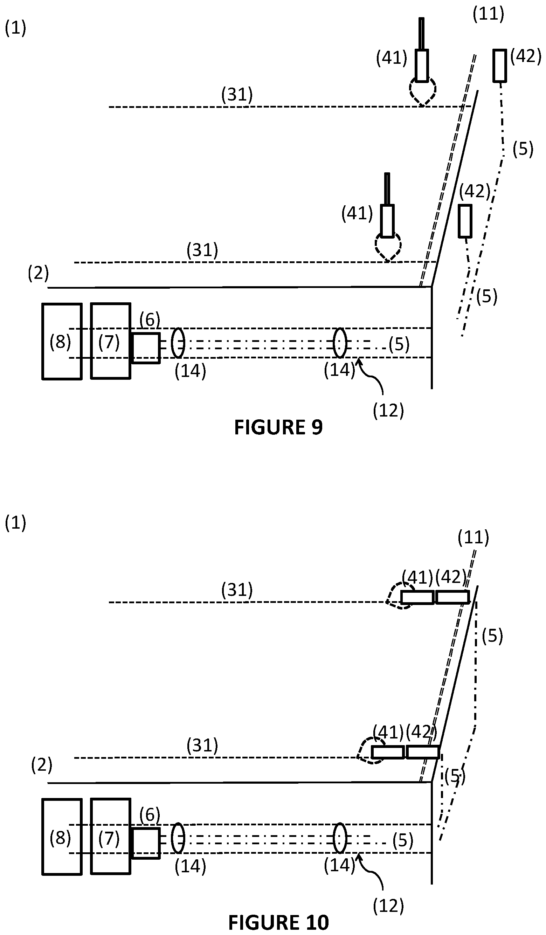

According to another aspect not representing the present invention (see **Figures 9** and **10**), the energy conducting connection between said conducting textile disposition (3) and said control device (7), comprising first connection means (4) adapted for conducting connection of a first and second end of said conducting textile disposition (3), and energy conducting disposition (5) adapted for connection of said first connection means (4) and second connection means (6) adapted for conducting connection of said energy conducting disposition (5) to said control device (7), whereby both of said first and second connection means (4, 6) are provided as pieces comprising respective conducting metallic parts. Moreover, said first connection means (4) are adapted so that they can be fixed to said conducting textiles (31) by means of a clamming type of connection.

It is further preferred when said conducting textiles (31) are united to respective conducting elements of said energy conducting disposition (5), by means of first connection means (4) provided in a region of said conducting textiles (31) next to said fixation means (11), preferentially respectively on the inward side with reference to the end of the top part of textile material support (2). As further represented in Figures 9 and 10, said first connection means (4) are advantageously provided as male-female type of connections, presenting a part of conducting textile connection (31) by means of crushing, and a conducting connection part. It is preferred when the removal of said connection by crushing requires the use of a tool. It is preferred when said first connection means (4) result on the top part, next to an end thereof.

Moreover, it is preferred when said energy conducting disposition (5) passes along at least part of the extension of said textile material support and is retained along at least part of a lateral part by a plurality of retention elements (14) associated with said textile material support (2) and configured in the form of a chain-link, or similar, so as to keep the plurality of conducting yarns included on said energy conduction disposition (5) in relative proximity between each other along at least part of respective extension.

It is preferred when said energy conducting disposition (5) is retained along at least part of its extension by a plurality of retention elements (14) associated with said textile material support (2) and configured in form of a chain-link, or similar, so as to keep the plurality of conducting yarns included on said energy conducting disposition (5) in relative proximity between each other along at least part of respective extension.

An electric connection of simple construction, but reliable is herewith advantageously provided, in particular resistant to the movements by an individual on said top part of textile material support (2) that presents said conducting textile disposition (3).

In the following, an alternative realisation not forming part of the present invention is described. The monitoring disposition (1) presents a textile material support (2) that comprises a first support element (21) presenting a conducting textile disposition (3) with first conducting textiles (31) developing along a first direction, and a second support element (22) presenting a textile disposition (3) with second conducting textiles (32) developing along a second direction orthogonal with relation to said first direction.

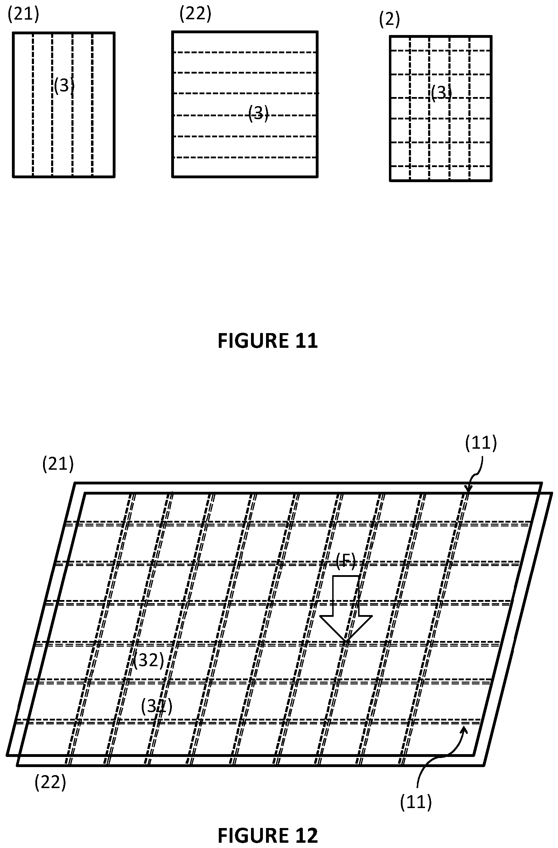

It is preferred when, as represented in **Figure 11**, said first and second support elements (21, 22) are initially produced separately, preferentially by means of a similar process and machine, and associated with each other by means of superposition so that there results a layered disposition and with a matrix-like configuration of respective first and second conducting textiles (31, 32) .

As represented in **Figure 12**, in the case there in an electric tension available at the ends of first and second conducting textiles (31, 32), the application of a force with a component transversal to the surface of the top part of said textile material support (2) thus results in the establishment of an electric contact in the intersecting zones.

As further represented, the conducting textiles (31, 32) are in this case retained in the textile material support (2) by fixing means (11) that develop along respective elongated extension. Moreover, said first connection means (4) would in this case be provided in the proximity of the retention surface - not represented -, as shall be promptly understood by the expert in the field.

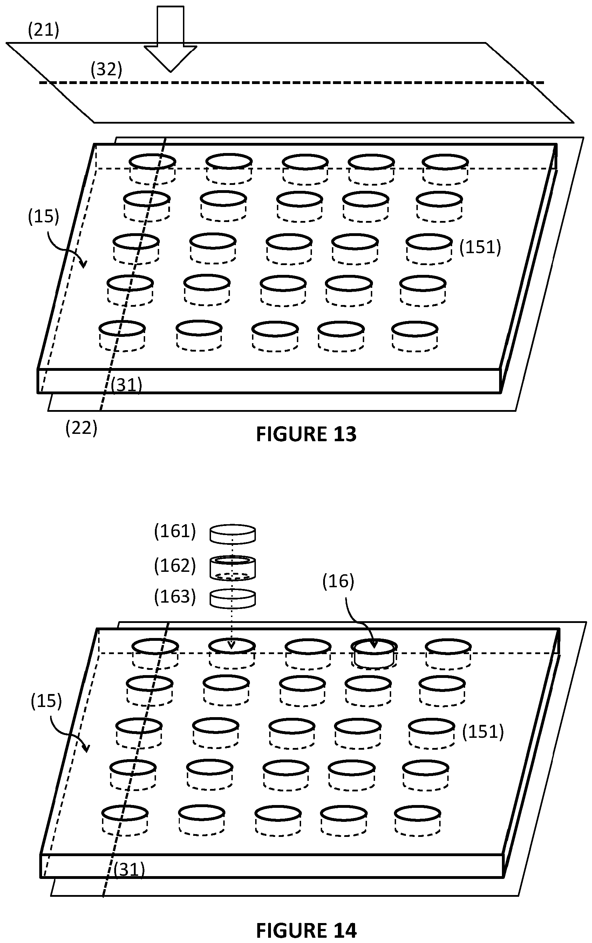

According to a realisation not forming part of the present invention represented in **Figure 13**, the monitoring disposition (1) further presents an electric insulating element (15) arranged between first and second support elements (21, 22) of said textile material support (2), whereby said electric insulating element (15) presents a similar shape to the superposed top parts of said first and second support elements, there is provided an electric insulating material, such as for example a synthetic material, with a foam-like structure or similar, and presents a plurality of crossing holes (151) provided in regions corresponding to the regions of intersection in superposition of said first and second conducting textiles (31, 32), so that provide and electric contact between these when said monitoring disposition (1) is submitted to a load long the direction of the gravity force.

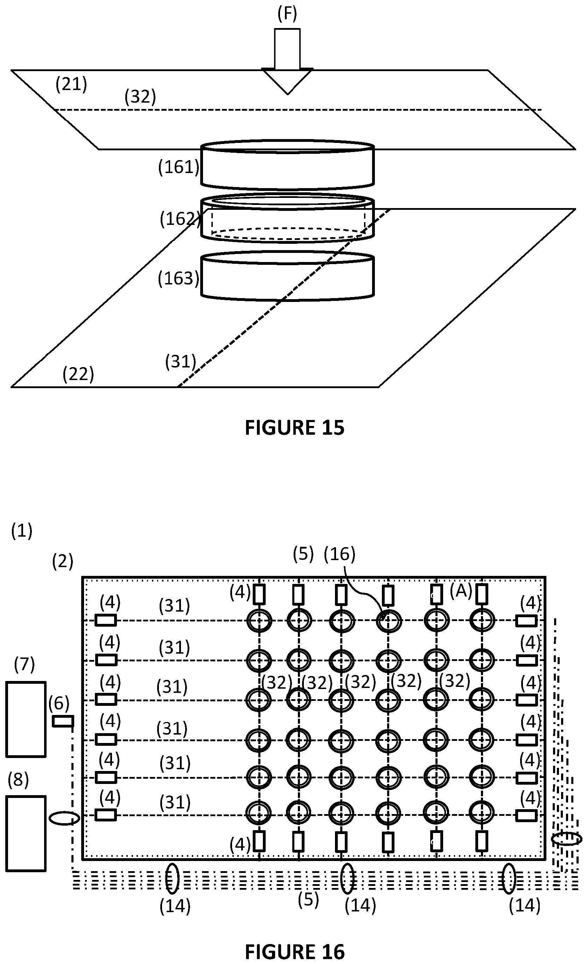

According to an example not representing the invention, represented in **Figures 14** and **15**, the monitoring disposition (1) presents an electric insulation element (15) arranged between first and second support elements (21, 22) of said textile material support (2), presenting a plurality of crossing holes (151), whereby each one of said crossing holes (151) presents a pressure-switch disposition (16) that comprises a first element (161) in an electric conducting material, with a foam-like structure or similar, presenting a thickness of up to 5 mm, and a distancing element (162) provided in a non-conducting material, preferentially with a foam-like structure, configured for example with a ring-like shape of similar, presenting a height of up to 5 mm.

It is further preferred when said pressure switch disposition (16) comprises a second conducting foam layer (163), in electric conducting material, with a foam-like structure or similar, presenting a thickness of up to 5 mm.

**Figure 16** shows a diagram of the set of said alternative realisation not forming part of the present invention.

In the case of this second embodiment, the control device (7) is adapted so that applies an electric tension to a first textile yarn (31) in a first direction, and measure the electric tensions available at that moment in each one of the textile yarns (2) along a second direction, repeating thereafter this cycle successively for each one of the remaining first textile yarns (31) along a first direction. As surely promptly understood by the expert in the technique, the application of a force upon the textile material support (2) shall produce a contact between said first textile yarn (31) and at least a second textile yarn (32), whereby said control device (7) is further adapted so that can reference the relative position of first and second textile yarns (31, 32) where there is measure an electric voltage resulting from contact between them.

The data measured by the control device (7) can be registered and related with other data measured in time, so as to establish relations with types of events and behaviour patterns.

## Claims

1. **Cover disposition (1)** for monitoring individuals in support surfaces of individuals, whereby said cover disposition (1) comprises:
- a textile material support (2) presenting a top part (21, 22) adapted for covering at least part of upwards-oriented surface of an object (A) that is adapted for supporting individuals;
- a conducting textile disposition (3) comprising a plurality of conducting textiles (31, 32) and associated with said textile material support (2);
- a control device (7) provided in energy conducting connection with said conduction textile disposition (3) and with an energy source (8),
whereby said textile material support (2) presents a top part (21, 22) that comprises at least part of said conducting textile disposition (3), and
wherein said textile material support (2) is provided so that it can be arranged over said object (A) so that said conducting textiles disposition (3) is substantially retained relative to said object (A), at least with relation to movements along a longitudinal extension of said object (A), and
wherein said control device (7) and electric connections between the latter and said conducting textile disposition (3) are associated with said textile material support (2) so that they are retained thereon, and
**characterized in that**
said electrical connections are provided so that the placement of said textile material support (2) upon said object (A) does not require establishing any electrical connection, and the removal of said textile material support (2) does not require disconnecting any electrical connection between said control device (7) and said conducting textile disposition (3), by providing the textile material support with a general shape so as to enclose said object (A) for supporting individuals inside thereof, and by providing said control device, energy source and all the energy conducting connections between said conducting textile disposition and said control device inside of the enclosure provided by said textile material support, so that said cover disposition does not present wire connections with the exterior thereof.

2. Disposition according to claim 1, **characterized in that** said textile material support (2) further presents at least one of:
- a general configuration at least partially closed so that it can at least partially collect said object (A) inside thereof underneath said top part (21, 22) and in a substantially fixed position with relation to said textile material support (2), and
- retention means (23) configured to retain said textile material support (2) in a substantially fixed position with relation to said object (A).

3. Disposition according to claims 1 or 2, **characterized in that** said textile material support (2) is provided with retention means (23) adapted so as to provide at least one of: retention and fixation, of said textile material support (2) on said object (A) for supporting individuals, so that said textile material support (2) can be placed and removed from said object (A) for supporting individuals.

4. Disposition according to any of claims 1 to 3, **characterized in that** said textile material support (2) is provided with a general shape of box, cylinder, sleeve type, configured to envelope at least most part of the exterior surface of the object (A) for supporting individuals, including of the type mattress, pillow, seat, and preferentially further a lateral part provided between said top part and base part, and a base part that does not present conducting textile disposition (3).

5. Disposition according to any of claims 1 to 4, **characterized in that** said textile material support (2) presents support connection means (12) adapted so as to provide a connection between at least two of said textile material support parts (2), such as for example of said top part (21, 22) to the remanding parts, along at least part of a perimeter region of said textile material support (2), so that said textile material support (2) can be opened, applied as envelope of a support element, and closed, and vice-versa.

6. Disposition according to any of claims 1 to 5, **characterized in that** said textile material support (2) presents support connection means (12) adapted so as to provide a connection between at least two of said textile material support parts (2), of the type manually removable connection means, including of continuous form, such as for example of the zip type, and of punctual form, such as for example push button.

7. Disposition according to any of previous claims 1 to 6, **characterized in that** said textile material support (2) presents support connection means (12) adapted so as to provide a connection between at least two of said parts of the textile material support (2), and to provide a guiding support to at least part of the extension of said energy conduction disposition (5), whereby said guiding support is provided as at least one of passageway chain-links, or passageway sheath, so as to contain said energy conduction disposition (5) in at least two regions, preferentially in a continuous extension, along at least part of a respective extension.

## Patentansprüche

1. Abdeckanordnung (1) zur Überwachung von Personen in Auflageflächen von Personen, wobei die Abdeckanordnung (1) umfasst:
- einen Träger (2) aus textilem Material, der einen oberen Teil (21, 22) aufweist, der geeignet ist, mindestens einen Teil der nach oben gerichteten Oberfläche eines Objekts (A) zu bedecken, das geeignet ist, Personen zu tragen;
- eine leitende Textilanordnung (3), die eine Vielzahl von leitenden Textilien (31, 32) umfasst und mit dem Träger (2) aus textilem Material verbunden ist;
- eine Steuervorrichtung (7), die in energieleitender Verbindung mit der leitenden Textilanordnung (3) und mit einer Energiequelle (8) steht,
wobei der Träger (2) aus textilem Material einen oberen Teil (21, 22) aufweist, der zumindest einen Teil der leitenden Textilanordnung (3) umfasst, und wobei der Träger (2) aus textilem Material so vorgesehen ist, dass er über dem Objekt (A) angeordnet werden kann, so dass die leitende Textilanordnung (3) im Wesentlichen relativ zu dem Objekt (A) gehalten wird, zumindest in Bezug auf Bewegungen entlang einer Längsausdehnung des Objekts (A), und
wobei die Steuervorrichtung (7) und die elektrischen Verbindungen zwischen dieser und der leitenden Textilanordnung (3) mit dem Träger (2) aus textilem Material verbunden sind, so dass sie darauf gehalten werden, und
**dadurch gekennzeichnet, dass**
die elektrischen Verbindungen so vorgesehen sind, dass das Anbringen des Trägers aus textilem Material (2) auf dem Objekt (A) keine Herstellung einer elektrischen Verbindung erfordert und das Entfernen des Trägers aus textilem Material (2) kein Trennen einer elektrischen Verbindung zwischen der Steuervorrichtung (7) und der leitenden Textilanordnung (3) erfordert, indem der Träger (2) aus textilem Material mit einer allgemeinen Form vorgesehen wird, um das Objekt (A) zum Tragen von Personen zu umschließen, und indem die Steuervorrichtung, die Energiequelle und alle energieleitenden Verbindungen zwischen der leitenden Textilanordnung und der Steuervorrichtung innerhalb der durch den Träger aus textilem Material gebildeten Umschließung vorgesehen werden, so dass die Abdeckanordnung keine Drahtverbindungen mit deren Außenseite aufweist.

2. Anordnung nach Anspruch 1, **dadurch gekennzeichnet, dass** der Träger (2) aus textilem Material außerdem mindestens eines der folgenden Merkmale aufweist:
- eine allgemeine Konfiguration, die zumindest teilweise geschlossen ist, so dass sie zumindest teilweise den Gegenstand (A) in ihrem Inneren unterhalb des oberen Teils (21, 22) und in einer im Wesentlichen festen Position in Bezug auf den Träger (2) aus textilem Material aufnehmen kann, und
- Haltemittel (23), die so konfiguriert sind, dass sie den Träger (2) aus textilem Material in einer im Wesentlichen festen Position in Bezug auf den Gegenstand (A) halten.

3. Anordnung nach Anspruch 1 oder 2, **dadurch gekennzeichnet, dass** der Träger (2) aus textilem Material mit Haltemitteln (23) versehen ist, die geeignet sind, mindestens eines von Folgendem zu gewährleisten: Halten und Fixieren des Trägers (2) aus textilem Material auf dem Objekt (A) zum Tragen von Personen, so dass der Träger (2) aus textilem Material auf dem Objekt (A) zum Tragen von Personen platziert und von diesem entfernt werden kann.

4. Anordnung nach einem der Ansprüche 1 bis 3, **dadurch gekennzeichnet, dass** der Träger (2) aus textilem Material eine allgemeine Form eines Kastens, eines Zylinders oder eines Hüllentyps aufweist, die so gestaltet ist, dass sie zumindest den größten Teil der Außenfläche des Objekts (A) zum Tragen von Personen, einschließlich des Typs Matratze, Kissen, Sitz, umhüllt, und vorzugsweise ferner einen seitlichen Teil, der zwischen dem oberen Teil und dem Basisteil vorgesehen ist, und einen Basisteil aufweist, der keine leitende Textilanordnung (3) aufweist.

5. Anordnung nach einem der Ansprüche 1 bis 4, **dadurch gekennzeichnet, dass** der Träger (2) aus textilem Material eine Trägerverbindungseinrichtung (12) aufweist, die so ausgebildet ist, dass sie eine Verbindung zwischen mindestens zwei der Teile des Trägers (2) aus textilem Material, wie z.B. dem Oberteil (21, 22) mit den übrigen Teilen, entlang mindestens eines Teils eines Umfangsbereichs des Trägers (2) aus textilem Material (2) herstellt, so dass der Träger (2) aus textilem Material geöffnet, als Umhüllung eines Trägerelements angebracht und geschlossen werden kann und umgekehrt.

6. Anordnung nach einem der Ansprüche 1 bis 5, **dadurch gekennzeichnet, dass** der Träger (2) aus textilem Material Verbindungsmittel (12) aufweist, die geeignet sind, eine Verbindung zwischen mindestens zwei der Teile des Trägers (2) aus textilem Material herzustellen, und zwar vom Typ manuell abnehmbare Verbindungsmittel, einschließlich von kontinuierlicher Form, wie zum Beispiel vom Typ Reißverschluss, und von punktueller Form, wie zum Beispiel Druckknopf.

7. Anordnung nach einem der vorhergehenden Ansprüche 1 bis 6, **dadurch gekennzeichnet, dass** der Träger (2) aus textilem Material Trägerverbindungsmittel (12) aufweist, die so angepasst sind, dass sie eine Verbindung zwischen mindestens zwei der Teile des Trägers (2) aus textilem Material bereitstellen und eine Führungsunterstützung für mindestens einen Teil der Ausdehnung der Energieleitungsanordnung (5) bereitstellen, wobei die Führungsunterstützung als mindestens eines von Durchgangskettengliedern oder als Durchgangsmantel vorgesehen ist, um die Energieleitungsanordnung (5) in mindestens zwei Bereichen, vorzugsweise in einer kontinuierlichen Ausdehnung, entlang mindestens eines Teils einer jeweiligen Ausdehnung zu enthalten.

## Revendications

1. Dispositif de couverture (1) pour surveiller les individus dans les surfaces d'appui des individus, ledit dispositif de couverture (1) comprenant :
- un support en matière textile (2) présentant une partie supérieure (21, 22) destinée à recouvrir au moins une partie de la surface orientée vers le haut d'un objet (A) destiné à soutenir des individus ;
- une disposition textile conductrice (3) comprenant une pluralité de textiles conducteurs (31, 32) et associée audit support en matière textile (2) ;
- un dispositif de commande (7) relié par conduction d'énergie à ladite disposition textile de conduction (3) et à une source d'énergie (8),
dans lequel ledit support en matière textile (2) présente une partie supérieure (21, 22) qui comprend au moins une partie de ladite disposition textile conductrice (3), et dans lequel ledit support en matière textile (2) est prévu pour être disposé sur ledit objet (A) de manière à ce que ladite disposition textile conductrice (3) soit sensiblement retenue par rapport audit objet (A), au moins en ce qui concerne les mouvements le long d'une extension longitudinale dudit objet (A), et
dans lequel ledit dispositif de commande (7) et les connexions électriques entre ce dernier et ladite disposition textile conductrice (3) sont associés audit support en matière textile (2) de sorte qu'ils y sont retenus, et
**caractérisée en ce que**
lesdites connexions électriques sont prévues pour que la mise en place dudit support en matière textile (2) sur ledit objet (A) ne nécessite pas l'établissement d'une connexion électrique, et que le retrait dudit support en matière textile (2) ne nécessite pas la déconnexion d'une connexion électrique entre ledit dispositif de commande (7) et ladite disposition textile conductrice (3), en dotant ledit textile de support en matière d'une forme générale permettant d'envelopper ledit objet (A) pour soutenir des individus à l'intérieur de celui-ci, et en dotant ledit dispositif de commande, la source d'énergie et toutes les connexions de conduction d'énergie entre ladite disposition textile conductrice et ledit dispositif de commande à l'intérieur de l'enceinte fournie par ledit textile de support en matière, de sorte que ladite disposition de couverture ne présente pas de connexions filaires avec l'extérieur de celle-ci.

2. Disposition selon la revendication 1, **caractérisée en ce que** ledit support en matière textile (2) présente en outre au moins l'un des éléments suivants :
- une configuration générale au moins partiellement fermée de manière à pouvoir recueillir au moins partiellement ledit objet (A) à l'intérieur de celui-ci sous ladite partie supérieure (21, 22) et dans une position sensiblement fixe par rapport audit support en matière textile (2), et
- des moyens de rétention (23) configurés pour retenir ledit support en matière textile (2) dans une position sensiblement fixe par rapport audit objet (A).

3. Disposition selon les revendications 1 ou 2, **caractérisée en ce que** ledit support en matière textile (2) est pourvu de moyens de rétention (23) adaptés pour assurer au moins l'une des fonctions suivantes : rétention et fixation dudit support en matière textile (2) sur ledit objet (A) pour soutenir des individus, de sorte que ledit support en matière textile (2) puisse être placé et retiré dudit objet (A) pour soutenir des individus.

4. Disposition selon l'une des revendications 1 à 3, **caractérisée en ce que** ledit support en matière textile (2) est pourvu d'une forme générale de type boîte, cylindre, manchon, configurée pour envelopper au moins la majeure partie de la surface extérieure de objet (A) pour soutenir des individus, y compris de type matelas, oreiller, siège, et préférentiellement en outre d'une partie latérale prévue entre ladite partie supérieure et la partie de base, et d'une partie de base qui ne présente pas de disposition textile conductrice (3).

5. Disposition selon l'une des revendications 1 à 4, **caractérisée en ce que** ledit support en matière textile (2) présente des moyens de connexion de support (12) adaptés pour assurer une connexion entre au moins deux desdites parties du support en matière textile (2), comme par exemple de ladite partie supérieure (21, 22) aux parties de remodelage, le long d'au moins une partie d'une région périmétrique dudit support en matière textile (2), de sorte que ledit support en matière textile (2) peut être ouvert, appliqué comme enveloppe d'un élément de support, et fermé, et vice-versa.

6. Disposition selon l'une des revendications 1 à 5, **caractérisée en ce que** ledit support en matière textile (2) présente des moyens de liaison au support (12) adaptés de manière à assurer une liaison entre au moins deux desdites parties du support en matière textile (2), de type moyens de liaison amovibles manuellement, y compris de forme continue, telle que par exemple de type fermeture à glissière, et de forme ponctuelle, telle que par exemple bouton poussoir.

7. Disposition selon l'une des revendications 1 à 6, **caractérisée en ce que** ledit support en matière textile (2) présente des moyens de connexion de support (12) adaptés pour assurer une connexion entre au moins deux desdites parties du support en matière textile (2), et pour fournir un support de guidage à au moins une partie de l'extension de ladite disposition de conduction d'énergie (5), ledit support de guidage est fourni sous la forme d'au moins un des maillons de passage ou d'une gaine de passage, de manière à contenir ladite disposition de conduction d'énergie (5) dans au moins deux régions, de préférence dans une extension continue, le long d'au moins une partie d'une extension respective.
